# EUROPÄISCHE PATENTSCHRIFT

(11) **EP 1 443 049 B1**
(45) Veröffentlichungstag und Bekanntmachung des Hinweises auf die Patenterteilung: **30.03.2005**
(21) Anmeldenummer: 03104546.1
(22) Anmeldetag: 04.12.2003
(51) Int. Cl.: C07D 491/10

(54) **Verfahren zur Herstellung von Ketalen**
Process for the preparation of ketals
Procédé de préparation de cétales

(30) Priorität: 01.02.2003 DE 10304055
(43) Veröffentlichungstag der Anmeldung: 04.08.2004
(73) Patentinhaber: Degussa AG, 40474 Düsseldorf (DE)
(72) Erfinder: Meyer, Oliver Dr., 48151 Münster (DE); Uhlenberg, Renate, 45657 Recklinghausen (DE); Korell, Michael Dr., Denville, 07834 (US)

(56) Entgegenhaltungen:
- DE-A- 2 203 533
- US-A- 3 940 401
- US-A- 3 963 730
- US-A- 4 250 312

## Beschreibung

Die Erfindung betrifft ein verbessertes Verfahren zur Herstellung von cyclischen und offenkettigen Ketalen insbesondere des Triacetonamins.

Die Ketalisierung von Triacetonamin (2,2,6,6-Tetramethyl-4-piperidon, TAA) mit ein- oder mehrwertigen Alkoholen zu den entsprechenden offenkettigen beziehungsweise cyclischen Ketalen ist im Prinzip aus der Literatur bekannt. So wird die Synthese diverser TAA-Ketale unter anderem beschrieben in DE 22 03 533, DE 23 53 538, CS 272 639, US 3 790 525, US 3 940 401, EP 0 222 512, EP 0 291 238 und EP 0 141 502. Als Katalysatoren werden dabei die dem Fachmann für diese Art von Reaktion bekannten Säuren wie wässrige Salzsäure, Schwefelsäure, Phosphorsäure oder diverse Sulfonsäuren (meist p-Toluolsulfonsäure) genannt. Da bei der Ketalisierung von TAA bereits ein Säureäquivalent für die Neutralisation der sekundären Aminfunktion verbraucht wird, muss anders als bei neutralen Verbindungen stets eine überstöchiometrische Mengen Säure eingesetzt werden. Ein geringer Überschuss an Katalysator ist für die Reaktion also in jedem Falle notwendig.

Des Weiteren finden sich in der Literatur Beispiele, bei denen nicht von TAA selbst, sondern von dessen Hydrochlorid ausgegangen wird (Monatsh. Chem. 93, 1962, 1090 - 1106; Scripta Fac. Sci. Nat. Univ. Masaryk. Brun., Vol. 23, 1993; JP 56 138 189; JP 56 025 185). Dieses TAA-Hydrochlorid wird dann analog zu den oben genannten Beispielen nach Zugabe einer Katalysatorsäure und dem jeweiligen Alkohol zum Ketal umgesetzt. Diese Verfahrensweise bedeutet jedoch einen zusätzlichen Synthese- und Isolierungsschritt, der für die großtechnische Produktion von TAA-Ketalen wenig vorteilhaft ist.

Darüber hinaus ist die Ketalisierung von TAA-Hydrochlorid beziehungsweise TAA-Sulfonsäuresalz auch durch Umsetzung mit einem Orthoester (EP 0 748 849) und durch Umketalisierung beschrieben worden (US 4 250 312, JP 55 092 386, EP 0 748 849).

Die Ketale des TAA können unter anderem als Stabilisatoren im Polymerbereich eingesetzt werden. Darüber hinaus sind aus ihnen durch Oxidation der sekundären Aminfunktion die entsprechenden N-Oxyl-Radikale zugänglich, die wiederum als Oxidationskatalysatoren, als Polymerisationsinhibitoren oder als Massenregulatoren bei Polymerisationen eingesetzt werden können.

Es bestand die Aufgabe, ein Verfahren zur Herstellung von offenkettigen oder cyclischen Ketalen insbesondere des Triacetonamins bereitzustellen, das die oben genannten Nachteile nicht aufweist und das insbesondere von einfachen Ausgangsverbindungen ausgeht, ohne zusätzliche Synthese- und Isolierungsschritte auskommt und sich problemlos in großtechnischem Maßstab durchführen lässt.

Es wurde überraschend gefunden, dass sich Triacetonamin mit Hydroxy-Derivaten mit einer oder mehreren Hydroxygruppen wie insbesondere ein- oder mehrwertige Alkohole, und mit gasförmigem Chlorwasserstoff umsetzen lässt, ohne dass dabei unerwünschte Nebenreaktionen wie zum Beispiel Chlorierungen der Hydroxygruppe in nennenswertem Maße stattfinden.

Gegenstand der Erfindung ist daher ein Verfahren zur Herstellung von Ketalen durch Umsetzung von Triacetonamin mit Hydroxy-Derivaten mit einer oder mehreren Hydroxygruppen und mit gasförmigem Chlorwasserstoff zu offenkettigen beziehungsweise cyclischen Ketalen.

Die Erfindung betrifft insbesondere Verfahren zur Herstellung von cyclischen und offenkettigen Ketalen des Triacetonamins der Formeln: 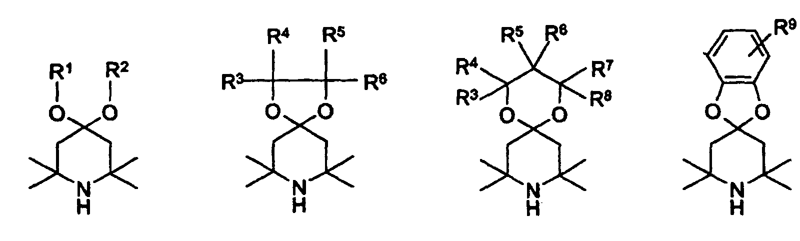 in denen R¹ und R² unabhängig voneinander eine Alkylgruppe mit 1 bis 10, vorzugsweise 1 bis 6, Kohlenstoffatomen, eine Alkoxyalkylgruppe oder eine Benzylgruppe,
R³, R⁴, R⁵, R⁶, R⁷ und R⁸ unabhängig voneinander Wasserstoff, eine Alkylgruppe mit 1 bis 10 Kohlenstoffatomen, eine Hydroxymethylengruppe (-CH₂OH), eine Esterfunktion (-CO₂R¹), eine Halogenmethylengruppe (-CH₂-X), eine Alkoxymethylengruppe (-CH₂OR¹), eine veresterte Alkoxymethylengruppe (-CH₂O₂CR¹), eine Alkoxyalkylgruppe, eine Benzylgruppe und
R⁹ entweder Wasserstoff, eine Alkylgruppe mit 1 bis 10 Kohlenstoffatomen, Halogen oder eine Alkoxygruppe (-OR¹) bedeutet.

Das erfindungsgemäße Verfahren ist dadurch gekennzeichnet, dass Triacetonamin und ein Hydroxy-Derivat mit einer oder mehreren Hydroxygruppen, insbesondere ein- oder mehrwertige Alkohole, mit gasförmigem Chlorwasserstoff umgesetzt wird. Eine weitere Katalysatorsäure ist dabei nicht erforderlich.

Bei Durchführung des erfindungsgemäßen Verfahrens zeigt sich völlig unerwartet, dass bereits während der Einleitung des gasförmigen Chlorwasserstoffs und somit vor der vollständigen Absättigung der sekundären Aminfunktion eine Umsetzung von Triacetonamin beziehungsweise des in situ gebildeten Triacetonamin-Hydrochlorids zum jeweiligen Triacetonamin-Ketal erfolgt. Obwohl also erst nach Ende der Chlorwasserstoff-Auftahme die Reaktionsmischung den für eine Ketalisierung notwendigen sauren pH-Wert aufweist, liegt der Triacetonamin-Umsatz zu diesem Zeitpunkt überraschenderweise für die meisten Triacetonamin-Ketale bereits bei 80 - 95 %. Es ist auch möglich, den gasförmigen Chlorwasserstoff überstöchiometrisch einzusetzen. Sofern gewünscht kann im Anschluss daran das bei der Ketalisierung gebildete Reaktionswasser vorzugsweise durch azeotrope Destillation aus dem System entfernt ("ausgekreist") und der Umsatz auf diese Weise vervollständigt werden.
Nach Neutralisation der Reaktionsmischung und entsprechender Aufarbeitung (zum Beispiel durch Destillation oder Filtration und Wäsche) erhält man die jeweiligen Triacetonamin-Ketale mit hoher Ausbeute und in hoher Reinheit.

Zur Erleichterung der Rührfähigkeit bei der Umsetzung kann dem Ansatz ein gegenüber Chlorwasserstoff inertes Lösemittel zugesetzt werden. Geeignet sind acyclische (aliphatische) Kohlenwasserstoffe wie zum Beispiel Heptan, cyclische (cycloaliphatische) Kohlenwasserstoffe wie zum Beispiel Cyclohexan oder Ethylcyclohexan und insbesondere aromatische Kohlenwasserstoffe wie zum Beispiel Toluol oder Xylol, die nach der Umsetzung auch als Schleppmittel für die Entfernung des bei der Reaktion entstandenen Wassers dienen können.

Die Reaktion wird in der Regel zwischen etwa 20 und etwa 150 °C, vorzugsweise zwischen 40 und 120 °C und ganz besonders bevorzugt zwischen 50 und 90 °C durchgeführt.

Die Reaktion wird vorzugsweise bei Normaldruck durchgeführt. Es ist aber auch möglich, gegebenenfalls die Reaktion bei leichtem Unterdruck (bezogen auf Normaldruck) oder Überdruck bis vorzugsweise 10 bar durchzuführen.

Das Verhältnis Triacetonamin zu einwertiger Hydroxyverbindung beträgt 1 : 2 bis 1: 8, vorzugsweise 1 : 2 bis 1 : 4.

Das Verhältnis Triacetonamin zu mehrwertiger (mindestens 2) Hydroxyverbindung beträgt 1 : 1 bis 1 : 4, vorzugsweise 1 : 1 bis 1 : 2.

Die Chlorwasserstoffinenge ist zum Ende des Einleitens überstöchiometrisch, d. h. mindestens 1 : 1 bezogen auf das Triacetonamin.

Als Hydroxyverbindung seien Alkohole, Hydroxycarbonsäuren, Hydroxyketone, Enole und Phenole, insbesondere aber ein- und mehrwertige Alkohole genannt. Als wichtigste Alkohole seien insbesondere 1,2-Ethandiol (1,2-Ethylenglykol), 1,2-Propandiol, 1,3-Propandiol, 1,2-Butandiol, 1,3-Butandiol, 1,2-Hexandiol, 1,2,3-Propantriol (Glycerin), Benzylalkohol und Brenzcatechin und Derivate genannt.

Die Umsetzung kann diskontinuierlich, semikontinuierlich oder kontinuierlich durchgeführt werden.

Wegen der Nachdosierung des gasförmigen Chlorwasserstoffs ist die bevorzugte Fahrweise eine diskontinuierliche bezüglich des Triacetonamins und des Hydroxy-Derivates und eine semikontinuierliche Fahrweise bezüglich des gasförmigen Chlorwasserstoffs.

Bei diskontinuierlicher Fahrweise beträgt die Reaktionszeit insgesamt ca. 60 Minuten bis ca. 3 Stunden inklusive der Zeit für das Einleiten des Chlorwasserstoffs.

Nach der Umsetzung wird mit einer üblichen basischen Verbindung neutralisiert. Als Basen können insbesondere Alkalihydroxide wie Natriumhydroxid oder Kaliumhydroxid, vorzugsweise als wässrige Lösung, aber auch Alkalicarbonate oder Alkoholate verwendet werden. Dabei hat der Einsatz von Alkali- oder Erdalkalialkoholaten, insbesondere von Natriummethylat, Natriumethylat, Kaliummethylat oder Kaliumethylat den erheblichen Vorteil, dass bei dieser Art der Synthese kein Abwasser anfällt. Die Alkoholate können in fester Form zum Beispiel als Pulver oder Granulat oder als alkoholische Lösung eingesetzt werden.

Die Aufarbeitung erfolgt in an sich bekannter Weise dadurch, dass feste (kristalline) Stoffe durch eine mechanische Trennoperation wie eine Filtration oder durch Zentrifugieren abgetrennt und gegebenenfalls gewaschen werden oder dass flüssige Produkte je nach Reinheitsanforderungen destillativ abgetrennt und gereinigt werden.

Zum Waschen eignen sich zum Beispiel Alkohole mit 1 bis 6 Kohlenstoffatomen ( wie z. B. Methanol, Ethanol, Isopropanol) oder Ether, sofern sie peroxidfrei sind, wie zum Beispiel Methyl-tert.-butylether oder Tetrahydrofuran.

Die Ketalisierung von Triacetonamin mit gasförmigem Chlorwasserstoff hat bedeutende Vorteile:
- Die Umsetzung von Triacetonamin mit dem Hydroxy-Derivat und Chlorwasserstoffgu kann im Vergleich zu einer Reaktion mit konzentrierter Salzsäure problemlos quantitativ durchgeführt werden. So zeigten die als Vergleichsversuche durchgeführten Umsetzungen von Triacetonamin mit Ethylenglykol und konzentrierter wässriger Salzsäure, dass sobald überschüssiges Wasser (und damit überschüssiger Chlorwasserstoff) ausgekreist worden war, die Reaktionsmischung einen pH-Wert von nur noch 4 - 5 erreicht hatte und der Umsatz bei ca. 80 - 85 % stehen blieb. Selbst durch Zugabe von p-Toluolsulfonsäure und wiederholter Wasserauskreisung konnte kein vollständiger Umsatz von Triacetonamin erreicht werden.
- Die Ketalisierung von Triacetonamin mit Chlorwasserstoffgas als Säure erfolgt im Vergleich zu einer Reaktion mit konzentrierter wässriger Salzsäure wesentlich schneller, da die überschüssige Wassermenge nicht erst wieder aus dem Reaktionsgemisch ausgekreist werden muss. Somit lässt sich durch das erfindungsgeinäße Verfahren im Vergleich zum Einsatz von konzentrierter wässriger Salzsäure eine wesentlich höhere Raum-Zeit-Ausbeute erreichen.
- Vergleichsexperimente haben gezeigt, dass es beim Einsatz von Schwefel- oder Phosphorsäure als Katalysator zu Bildung von erheblichen Mengen an Nebenprodukten kommt, was vermutlich auf eine Zersetzung von Triacetonamin zurückzuführen ist. Diese Nebenproduktbildung wird durch das Verfahren gemäß vorliegender Erfindung vermieden.
- Als Abfall fällt bei dem erfindungsgemäßen Verfahren nach Neutralisation mit einer Base nur das entsprechende Chloridsalz an (zum Beispiel Natriumchlorid), welches wesentlich besser (kostengünstiger) entsorgt werden kann als andere Salze wie zum Beispiel Natriumsulfat, Natriumphosphat oder die entsprechenden Sulfonsäuresalze.
- Chlorwasserstoffgas ist kostengünstig, gut dosierbar und im technischen Maßstab oft per Leitung verfügbar. Dadurch bedingt lässt sich Chlorwasserstoffgas einfach handhaben und verursacht praktisch keine Lagerkosten.

### Beispiele

### Beispiel 1

### Darstellung von 2-(Hydroxymethyl)-7,7,9,9-tetramethyl-1,4-dioxa-8-azaspiro[4.5]decan

155 g Triacetonamin wurden zusammen mit 184 g Glycerin in 500 ml Toluol vorgelegt. Anschließend wurde über ein Tauchrohr Chlorwasserstoffgas in den Reaktor eingeleitet, wobei die Sumpftemperatur unter 80 °C gehalten wurde. Sobald die Chlorwasserstoff-Aufnahme deutlich nachließ, wurde das Reaktionsgemisch auf Rückflusstemperatur erhitzt und entstandenes Reaktionswasser am Wasserabscheider ausgekreist.

Sobald vollständiger TAA-Umsatz erreicht war, ließ man das Reaktionsgemisch abkühlen und stellte durch Zugabe von 200 g 25 %iger NaOH-Lösung und 47 g Wasser einen pH-Wert von 11 ein. Das ausgefallene Produkt wurde über eine Glasfilternutsche abgesaugt, nacheinander mit Wasser und Isopropanol gewaschen und im Vakuum getrocknet. Man erhielt das Produkt als weißen, rieselfähigen Feststoff.

| | |
|---|---|
| Ausbeute | 175 g (76 %) |
| Schmelzpunkt. | 136 - 138 °C |

### Beispiel 2:

### Darstellung von 7,7,9,9-Tetramethyl-1,4-dioxa-8-azaspiro[4.5]decan

248 g Triacetonamin wurden zusammen mit 199 g Ethylenglykol in 500 ml Toluol vorgelegt. Anschließend wurde über ein Tauchrohr Chlorwasserstoffgas in den Reaktor eingeleitet, wobei die Sumpftemperatur unter 80 °C gehalten wurde. Sobald die Chlorwasserstoff-Aufnahme deutlich nachließ, wurde das Reaktionsgemisch auf Rückflusstemperatur erhitzt und das entstandene Reaktionswasser am Wasserabscheider ausgekreist.

Sobald vollständiger TAA-Umsatz erreicht war, ließ man das Reaktionsgemisch abkühlen und stellte durch Zugabe von 303 g 25 %iger NaOH-Lösung und 76 g Wasser einen pH-Wert von 11 ein. Nach Trennung der Phasen wurde die wässrige Phase 3 mal mit Toluol extrahiert. Die vereinigten organischen Phasen wurden fraktioniert im Vakuum destilliert.

| | |
|---|---|
| Ausbeute | 217 g (90 %) |
| Siedepunkt | 83 °C/5 mbar |

### Beispiel 3:

### Darstellung von 2-Butyl-7,7,9,9-tetramethyl-1,4-dioxa-8-azaspiro[4.5]decan

248 g Triacetonamin wurden zusammen mit 378 g 1,2-Hexandiol in 500 ml Toluol vorgelegt. Anschließend wurde über ein Tauchrohr Chlorwasserstoffgas in den Reaktor eingeleitet, wobei die Sumpftemperatur unter 80 °C gehalten wurde. Sobald die Chlorwasserstoff-Aufnahme deutlich nachließ, wurde das Reaktionsgemisch auf Rückflusstemperatur erhitzt und das entstandene Reaktionswasser am Wasserabscheider ausgekreist.

Sobald vollständiger TAA-Umsatz erreicht war, ließ man das Reaktionsgemisch abkühlen und stellte durch Zugabe von 303 g 25 %iger NaOH-Lösung und 76 g Wasser einen pH-Wert von 11 ein. Nach Trennung der Phasen wurde die wässrige Phase 3 mal mit Toluol extrahiert. Die vereinigten organischen Phasen wurden fraktioniert im Vakuum destilliert.

| | |
|---|---|
| Ausbeute | 322 g (79 %) |
| Siedepunkt | 92 °C/0,5 mbar |

### Beispiel 4:

### Darstellung von 8,8,10,10-Tetramethyl-1,5-dioxa-9-azaspiro[5.5]undecan

248 g Triacetonamin wurden zusammen mit 243 g 1,3-Propandiol in 500 ml Toluol vorgelegt. Anschließend wurde über ein Tauchrohr Chlorwasserstoffgas in den Reaktor eingeleitet, wobei die Sumpftemperatur unter 80 °C gehalten wurde. Sobald die Chlorwasserstoff-Aufnahme deutlich nachließ, wurde das Reaktionsgemisch auf Rückflusstemperatur erhitzt und das entstandene Reaktionswasser am Wasserabscheider ausgekreist.

Sobald vollständiger TAA-Umsatz erreicht war, ließ man das Reaktionsgemisch abkühlen und stellte durch Zugabe von 303 g 25 %iger NaOH-Lösung und 76 g Wasser einen pH-Wert von 11 ein. Nach Trennung der Phasen wurde die wässrige Phase 3 mal mit Toluol extrahiert. Die vereinigten organischen Phasen wurden fraktioniert im Vakuum destilliert.

| | |
|---|---|
| Ausbeute | 287 g (84 %) |
| Siedepunkt | 72 °C/0,5 mbar |

### Vergleichsbeispiel 1:

### Darstellung von 8,8,10,10-Tetramethyl-1,5-dioxa-9-azaspiro[5.5]undecan; Einsatz von Schwefelsäure

155 g Triacetonamin wurden zusammen mit 124 g Ethylenglykol in 800 ml Toluol vorgelegt. Anschließend wurde soviel Schwefelsäure (106 g) hinzugegeben, dass die Reaktionsmischung beim Test mit einem angefeuchteten Indikatorpapier einen sauren pH-Wert (≤ 2) anzeigte. Das Reaktionsgemisch wurde auf Rückflusstemperatur erhitzt und entstandenes Reaktionswasser am Wasserabscheider ausgekreist. Nach ca. 45 Minuten fielen große Mengen eines in Wasser nicht löslichen Feststoffs aus, die sich als klebrige Anbackungen an Kolben und Rührer festsetzten.

### Vergleichsbeispiel 2:

### Darstellung von 8,8,10,10-Tetramethyl-1,5-dioxa-9-azaspiro[5.5]undecan; Einsatz von Phosphorsäure

77 g Triacetonamin wurden zusammen mit 47 g Ethylenglykol in 240 g Toluol vorgelegt. Anschließend wurde soviel Phosphorsäure (63 g) hinzugegeben, dass die Reaktionsmischung beim Test mit einem angefeuchteten Indikatorpapier einen sauren pH-Wert (≤ 2) anzeigte. Das Reaktionsgemisch wurde auf Rückflusstemperatur erhitzt und entstandenes Reaktionswasser am Wasserabscheider ausgekreist. Nach ca. 3 Stunden lagen laut Gaschromatogramm weniger als 10 % des gewünschten Produkts vor. Statt dessen hatten sich mehrere Nebenkomponenten gebildet, die jedoch nicht weiter untersucht wurden.

### Vergleichsbeispiel 3:

### Darstellung von 8,8,10,10-Tetramethyl-1,5-dioxa-9-azaspiro[5.5]undecan; Einsatz von konzentrierter wässriger Salzsäure

93 g Triacetonamin wurden zusammen mit 75 g Ethylenglykol und 65 g konzentrierter Salzsäure in 190 ml Toluol vorgelegt. Das Reaktionsgemisch wurde auf Rückflusstemperatur erhitzt und das entstandene Reaktionswasser am Wasserabscheider ausgekreist. Es zeigte sich, dass der pH-Wert des Sumpfes von zunächst 1 auf 4 - 5 fiel. Sobald dieser pH-Wert erreicht war, blieb der Umsatz bei ca. 80 - 85 % stehen. Nach Zugabe von p-Toluolsulfonsäure als Katalysator und erneuter Wasserauskreisung konnte der Umsatz zwar geringfügig gesteigert werden, allerdings wurde auch nach dreifacher Zugabe und Wasserauskreisung kein quantitativer TAA-Umsatz erreicht.

## Patentansprüche

1. Verfahren zur Ketalisierung von Triacetonamin,
**dadurch gekennzeichnet,**
**dass** Triacetonamin und ein Hydroxy-Derivat mit einer oder mehreren Hydroxygruppen mit gasförmigem Chlorwasserstoff zum offenkettigen beziehungsweise cyclischen Ketal umgesetzt werden.

2. Verfahren nach Anspruch 1,
**dadurch gekennzeichnet,**
**dass** man die Ketalisierung in Gegenwart eines Lösemittels durchführt.

3. Verfahren nach einem der vorhergehenden Ansprüche,
**dadurch gekennzeichnet,**
**dass** als Lösemittel ein acyclischer, ein cyclischer oder ein aromatischer Kohlenwasserstoff eingesetzt wird.

4. Verfahren nach einem der vorhergehenden Ansprüche,
**dadurch gekennzeichnet,**
**dass** als Lösemittel Heptan, Cyclohexan, Ethylcyclohexan, Toluol oder Xylol eingesetzt wird.

5. Verfahren nach einem der vorhergehenden Ansprüche,
**dadurch gekennzeichnet,**
**dass** die Reaktion zwischen 20 °C und 150 °C, vorzugsweise zwischen 50 °C und 90 °C durchgeführt wird.

6. Verfahren nach einem der vorhergehenden Ansprüche,
**dadurch gekennzeichnet,**
**dass** man entstandenes Reaktionswasser aus der Reaktionsmischung auskreist.

7. Verfahren nach einem der vorhergehenden Ansprüche,
**dadurch gekennzeichnet,**
**dass** man Triacetonamin und ein Hydroxy-Derivat mit einer Hydroxygruppe im Verhältnis 1 : 2 - 8, vorzugsweise 1 : 2 - 4, einsetzt.

8. Verfahren nach einem der vorhergehenden Ansprüche,
**dadurch gekennzeichnet,**
**dass** man Triacetonamin und ein Hydroxy-Derivat mit mindestens zwei Hydroxygruppen im Verhältnis 1 : 1 - 4, vorzugsweise 1 : 1 - 2, einsetzt.

9. Verfahren nach einem der vorhergehenden Ansprüche,
**dadurch gekennzeichnet,**
**dass** es sich bei den Hydroxy-Derivaten um ein- oder mehrwertige Alkohole handelt.

10. Verfahren nach Anspruch 9,
**dadurch gekennzeichnet,**
**dass** als Hydroxy-Derivat Ethylenglykol oder Glycerin eingesetzt wird.

11. Verfahren nach einem der vorhergehenden Ansprüche,
**dadurch gekennzeichnet,**
**dass** überstöchiometrische Mengen Chlorwasserstoff eingesetzt werden.

12. Verfahren nach einem der vorhergehenden Ansprüche,
**dadurch gekennzeichnet,**
**dass** die Umsetzung diskontinuierlich durchgeführt wird, wobei der gasförmige Chlorwasserstoff nachdosiert wird.

13. Verfahren nach einem der vorhergehenden Ansprüche,
**dadurch gekennzeichnet,**
**dass** die Neutralisation des Reaktionsgemisches mit einem Alkali- oder Erdalkalialkoholat erfolgt.

14. Verfahren nach einem der vorhergehenden Ansprüche,
**dadurch gekennzeichnet,**
**dass** die Neutralisation des Reaktionsgemisches mit Natriummethylat, Natriumethylat, Kaliummethylat oder Kaliumethylat jeweils als Pulver oder in Form einer alkoholischen Lösung erfolgt.

15. Verfahren nach einem der Ansprüche 1 bis 11, 13 und 14 ,
**dadurch gekennzeichnet,**
**dass** die Umsetzung kontinuierlich durchgeführt wird.

## Claims

1. A process for ketalizing triacetonamine,
**characterized in that**
triacetonamine and a hydroxyl derivative having one or more hydroxyl groups are reacted with gaseous hydrogen chloride to give the open-chain or cyclic ketal.

2. A process according to claim 1,
**characterized in that**
the ketalization is carried out in the presence of a solvent.

3. A process according to any one of the preceding claims,
**characterized in that**
the solvent used is an acyclic, a cyclic or an aromatic hydrocarbon.

4. A process according to any one of the preceding claims,
**characterized in that**
the solvent used is heptane, cyclohexane, ethylcyclohexane, toluene or xylene.

5. A process according to any one of the preceding claims,
**characterized in that**
the reaction is carried out from 20°C to 150°C, preferably from 50°C to 90°C.

6. A process according to any one of the preceding claims,
**characterized in that**
water of reaction formed is removed from the reaction mixture.

7. A process according to any one of the preceding claims,
**characterized in that**
triacetonamine and a hydroxyl derivative having one hydroxyl group are used in a ratio of 1:2-8, preferably 1:2-4.

8. A process according to any one of the preceding claims,
**characterized in that**
triacetonamine and a hydroxyl derivative having at least two hydroxyl groups are used in a ratio of 1:1-4, preferably 1:1-2.

9. A process according to any one of the preceding claims,
**characterized in that**
the hydroxyl derivative is mono- or polyhydric alcohol.

10. A process according to claim 9,
**characterized in that**
the hydroxyl derivative used is ethylene glycol or glycerol.

11. A process according to any one of the preceding claims,
**characterized in that**
superstoichiometric amount of hydrogen chloride is used.

12. A process according to any one of the preceding claims,
**characterized in that**
the reaction is carried out batchwise, and the gaseous hydrogen chloride is added subsequently.

13. A process according to any one of the preceding claims,
**characterized in that**
the reaction mixture is neutralized using an alkali metal alkoxide or alkaline earth metal alkoxide.

14. A process according to any one of the preceding claims, **characterized in that** the reaction mixture is neutralized using sodium methoxide, sodium ethoxide, potassium methoxide or potassium ethoxide, each as a powder or in the form of an alcoholic solution.

15. A process according to any one of claims 1 to 11, 13 and 14,
**characterized in that** the reaction is carried out continuously.

## Revendications

1. Procédé de cétalisation de triacétonamine,
**caractérisé en ce qu'**
on convertit la triacétonamine et un dérivé hydroxy comportant un ou plusieurs groupes hydroxy avec du chlorure d'hydrogène gazeux en un cétal à chaîne ouverte ou cyclique.

2. Procédé selon la revendication 1,
**caractérisé en ce que**
on réalise la cétalisation en présence d'un solvant.

3. Procédé selon l'une quelconque des revendications précédentes,
**caractérisé en ce que**
comme solvant, on met en oeuvre un hydrocarbure acyclique, cyclique ou aromatique.

4. Procédé selon l'une quelconque des revendications précédentes,
**caractérisé en ce que**
comme solvant, on met en oeuvre de l'heptane, du cycloheptane, de l'éthylcyclohexane, du toluène ou du xylène.

5. Procédé selon l'une quelconque des revendications précédentes,
**caractérisé en ce que**
la réaction s'effectue entre 20°C et 150°C, de préférence entre 50°C et 90°C.

6. Procédé selon l'une quelconque des revendications précédentes,
**caractérisé en ce que**
l'eau de réaction produite est extraite du mélange réactionnel.

7. Procédé selon l'une quelconque des revendications précédentes,
**caractérisé en ce qu'**
on met en oeuvre de la triacétonamine et un dérivé hydroxy avec un groupe hydroxy dans un rapport de 1 :2 à 1 : 8, de préférence de 1 : 2 à 1 : 4.

8. Procédé selon l'une quelconque des revendications précédentes,
**caractérisé en ce qu'**
on met en oeuvre de la triacétonamine et un dérivé hydroxy comportant au moins deux groupes hydroxy dans un rapport de 1 / 1 à 4, de préférence de 1 : 1 à 1 : 2.

9. Procédé selon l'une quelconque des revendications précédentes,
**caractérisé en ce que**
les dérivés hydroxy sont des alcools mono ou polyvalents.

10. Procédé selon la revendication 9,
**caractérisé en ce que**
comme dérivé hydroxy, on met en oeuvre de l'éthylène glycol ou du glycérol.

11. Procédé selon l'une quelconque des revendications précédentes,
**caractérisé en ce qu'**
on met en oeuvre des quantités sur-stoechiométriques de chlorure d'hydrogène.

12. Procédé selon l'une quelconque des revendications précédentes,
**caractérisé en ce qu'**
on réalise la conversion en discontinu, le chlorure d'hydrogène gazeux étant ajouté ensuite.

13. Procédé selon l'une quelconque des revendications précédentes,
la neutralisation du mélange réactionnel est réalisée avec un alcoolate alcalin ou alcalino-terreux.

14. Procédé selon l'une quelconque des revendications précédentes,
**caractérisé en ce qu'**
on réalise la neutralisation du mélange réactionnel avec du méthylate de sodium, de l'éthylate de sodium, du méthylate de potassium ou de l'éthylate de potassium chacun sous forme de poudre ou d'une solution alcoolique.

15. Procédé selon l'une quelconque des revendications 1 à 11, 13 et 14,
**caractérisé en ce qu'**
on réalise la conversion en continu.
